# EUROPÄISCHE PATENTSCHRIFT

(11) **EP 1 679 050 B1**
(45) Veröffentlichungstag und Bekanntmachung des Hinweises auf die Patenterteilung: **16.07.2008**
(21) Anmeldenummer: 05022720.6
(22) Anmeldetag: 18.10.2005
(51) Int. Cl.: A61F 2/40

(54) **Inverse Schultergelenk-Endoprothese mit einteiligem Mittelteil**
Inverted shoulder joint endoprosthesis with one-piece middle part
Endoprothèse inversée de l'articulation de l'épaule avec pièce centrale monobloc

(30) Priorität: 11.01.2005 DE 102005001255
(43) Veröffentlichungstag der Anmeldung: 12.07.2006
(73) Patentinhaber: Mathys AG Bettlach, 2544 Bettlach (CH)
(72) Erfinder: Dallmann, Frank, 04626 Schmölln (DE)
(74) Vertreter: Körfer, Thomas

(56) Entgegenhaltungen:
- EP-A- 0 299 889
- EP-A- 1 064 890
- EP-A- 1 166 724
- EP-A- 1 269 941
- EP-A- 1 488 764
- DE-A- 10 037 504
- DE-C- 10 119 079
- DE-C- 10 123 517
- FR-A- 2 579 454
- FR-A- 2 704 747
- FR-A- 2 766 356
- US-A- 6 022 509

## Beschreibung

Die Erfindung geht aus von einer Schultergelenk-Endoprothese zur endoprothetischen Versorgung des menschlichen oder tierischen Schultergelenks nach dem Oberbegriff des Anspruchs 1.

Beispielsweise ist aus der EP 1 093 777 A2 eine Schulterendoprothese mit einem Gelenkkopf, mit einem Mittelteil, an dem der Gelenkkopf befestigbar ist, und mit einem Schaftteil, das ebenfalls an dem Mittelteil befestigbar ist, bekannt. Das Mittelteil ist gegenüber dem Schaftteil längs dessen Schaftachse und in seiner Drehlage um die Schaftachse einstellbar. Das Mittelteil weist eine Bohrung auf und ist mittels eines Spannelements in dieser Bohrung festspannbar.

Weiterhin ist aus der EP 0 299 889 B1 eine totale Schultergelenk-Endoprothese der inversen Bauart bekannt. Sie umfaßt ein glenoides Element, gebildet durch ein vorstehendes Kugelstück und dazu bestimmt, an der Gelenkpfanne des Schulterblattes befestigt zu werden, sowie ein Oberarmelement, gebildet durch einen Kugelnapf gleichen Durchmessers wie das vorstehende Kugelstück, das das glenoide Element bildet, wobei es mittels seiner Außenfläche fest an einer Stange sitzt, die dazu bestimmt ist, in die Oberarmdiaphyse gesteckt und nach Resektion des oberen Endes des Oberarmknochens darin befestigt zu werden.

Die europäische Patentschrift EP 1 064 890 A1 geht aus von einer inversen Schulterprothese, die einen in der Diaphyse des Humerus verankerbaren Schaft mit einem direkt daran angeschlossenen Mittelteil mit einem Napf, welcher die Gelenkpfanne nachbildet, aufweist. Das Mittelteil dieser Schulterprothese weist keine Ausnehmung zur Ausbildung einer Steckverbindung mit dem Schaft auf, da das untere Ende des Mittelteils und das obere Ende des Schafts mit ihren Stirnflächen aneinander gelegt sind.

Nachteilig an bekannten inversen Schultergelenk-Endoprothesen wie der oben beschriebenen ist dabei insbesondere, daß die meisten Materialkombinationen von Metall für die Basiskomponenten und einem Kunststoff für die Gelenkpfanne zu häufigen Schadensfällen führen. In der Folge muß der Patient nochmals operiert werden, um das Implantat auszutauschen.

Problematisch bei den bekannten inversen Schultergelenk-Endoprothesen ist dabei insbesondere der relativ wenig verschleißresistente Werkstoff Polyethylen für die Gelenkpfanne. Der häufig massive Partikelabrieb führt unabhängig von der Gefahr der Lockerung des Pfannen-Inlays zu aseptischen Lockerungsreaktionen der gesamten Endoprothese.

Aufgabe der Erfindung ist es demnach, eine mit dem glenoiden Element zusammenwirkende Gleitfläche so auszubilden, daß es nicht zu Schadensfällen durch Ablösung der Gleitfläche kommen kann. Der gewählte Werkstoff sollte dabei wesentlich verschleißresistenter als das bisher eingesetzte Polyethylen sein.

Die Aufgabe wird durch die kennzeichnenden Merkmale des Anspruchs 1 in Verbindung mit den gattungsbildenden Merkmalen gelöst.

Vorteilhafterweise ist das die Gelenkpfanne bildende Mittelteil dabei entweder einstückig aus einem geeigneten Material gefertigt oder besteht aus einer mit einem geeigneten Material nicht-lösbar umbauten Basis vorzugsweise aus Metall, welche jeweils mit einem Schaft verbindbar ist.

Schadensfälle durch Ablösen einer lösbar mit der Basis verbindbaren Gleitfläche werden somit ausgeschlossen und die Gefahr des Komplettausfalls oder der aseptischen Lockerung aufgrund massiven Abriebs der Gelenkpfanne durch die Wahl eines verschleißresistenteren Werkstoffes reduziert. Weiterhin ist eine einfache Handhabung durch den Operateur sowie eine Vereinfachung der Lagerhaltung gegeben, da das Mittelteil auf einen bereits in situ vorhandenen Schaft aufsetzbar ist.

Weitere vorteilhafte Ausgestaltungen der Erfindung sind in den Unteransprüchen gekennzeichnet.

Ausführungsbeispiele der Erfindung werden im folgenden anhand schematischer Darstellungen in der Zeichnung näher erläutert. In der Zeichnung zeigen:
- Fig. 1A-C: je eine perspektivische, eine seitliche und eine geschnittene Darstellung der Komponenten eines ersten Ausführungsbeispiels einer erfindungsgemäß ausgestalteten Schultergelenk-Endoprothese,
- Fig. 2A-C: je eine perspektivische, eine seitliche und eine geschnittene Darstellung der erfindungsgemäß ausgestalteten Schultergelenk-Endoprothese gemäß Fig. 1A-C in montiertem Zustand,
- Fig. 3A-B: eine perspektivische Darstellung und eine Ansicht von distal auf ein zweites Ausführungsbeispiel eines Mittelteils einer erfindungsgemäß ausgestalteten Schultergelenk-Endoprothese, und
- Fig. 4A-B: eine Ansicht von distal und eine Schnittdarstellung eines dritten Ausführungsbeispiel eines Mittelteils einer erfindungsgemäß ausgestalteten Schultergelenk-Endoprothese.

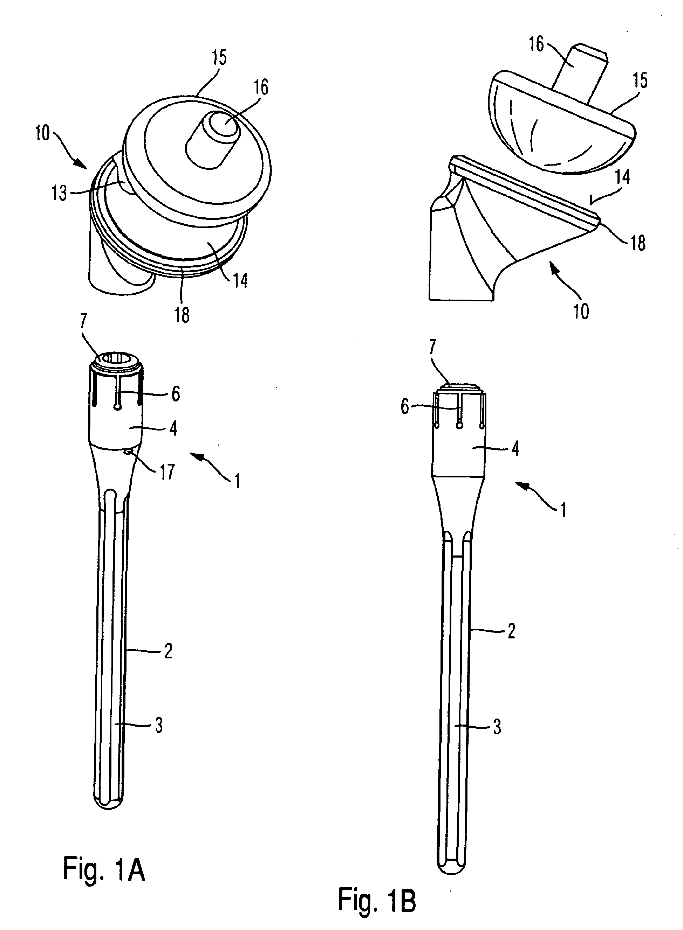

Fig. 1A bis 1C zeigt in verschiedenen Ansichten die Komponenten eines ersten Ausführungsbeispiels einer erfindungsgemäß ausgestalteten Schultergelenk-Endoprothese 1 in nichtmontiertem Zustand. Die erfindungsgemäß ausgestaltete Schultergelenk-Endoprothese 1 umfaßt einen Schaft 2, welcher in einen frakturierten oder vorbereitend zu resezierenden, nicht weiter dargestellten Oberarmknochen bzw. Humerus eingesetzt wird. Der Schaft 2 ist so gestaltet, daß er in einer Markhöhle des Humerus (Oberarmdiaphyse) verstemmt oder einzementiert werden kann. Der Schaft 2 kann dabei Längsrippen 3 aufweisen, um eine optimale Befestigung und Rotationssicherung in der Markhöhle zu erreichen.

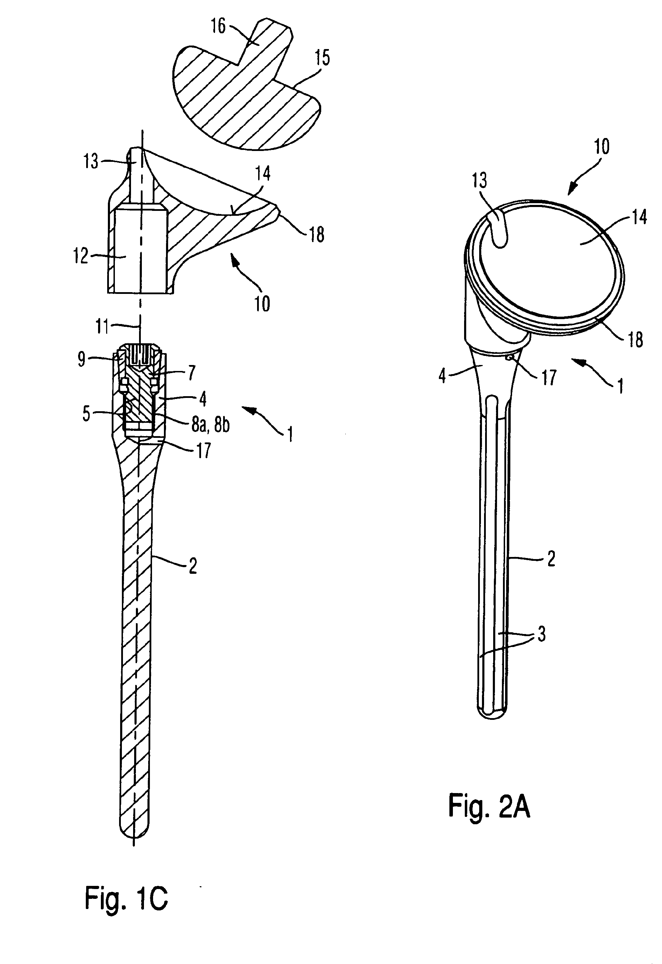

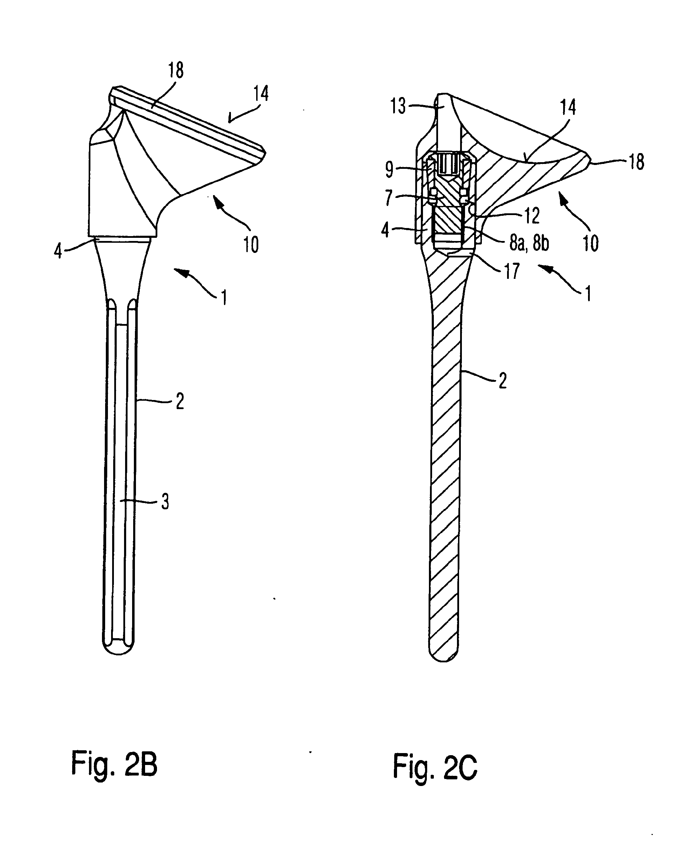

An seinem proximalen Ende weist der Schaft 2 einen radial erweiterten Bereich 4 auf, welcher hohlzylindrisch mit einer Ausnehmung 5 ausgebildet und mit Schlitzen 6 versehen ist, die über den Umfang des radial erweiterten Bereichs 4 verteilt angeordnet sind. In die Ausnehmung 5 ist ein verdrehbarer Einsatz 7 eingesetzt, welcher mit einem Gewinde 8a in ein korrespondierendes Gewinde 8b des radial erweiterten Bereichs 4 eingreift. Ein im Querschnitt keilförmiger Ringeinsatz 9 überträgt bei axialer Verdrehung des Einsatzes 7 durch ein geeignetes Werkzeug eine spreizende Kraft auf den radial erweiterten Bereich 4, so daß der Umfang des Bereichs 4 in radialer Richtung weiter vergrößert wird. In der Folge verspreizt sich der radial verbreiterte Bereich 4 in einem auf den Schaft 2 aufsetzbaren und auf dem radial erweiterten Bereich 4 verschieblichen Mittelteil 10. Die Situation in montiertem Zustand der Komponenten ist dabei in den Fig. 2A bis 2C in gleicher Darstellung wie in den Fig. 1A bis 1D gezeigt.

Das Mittelteil 10 ist vorzugsweise gekröpft relativ zu einer Längsachse 11 des Schaftes 2 ausgebildet. Es weist eine Ausnehmung 12 auf, welche der Aufnahme des radial verbreiterten Bereichs 4 des Schaftes 2 dient. Die Ausnehmung 12 setzt sich in proximaler Richtung mit einem verringerten Durchmesser als Montagebohrung 13 fort. Durch die Montagebohrung 13 wird das Werkzeug zum Verdrehen des Einsatzes 7 zur Verspreizung der Komponenten Schaft 2 und Mittelteil 10 eingeführt. Aus Fig. 2C ist ersichtlich, wie die beiden Komponenten zusammengesetzt sind und wie das nicht weiter dargestellte Werkzeug durch die Montagebohrung 13 des Mittelteils 10 einführbar ist.

Weiterhin weist das Mittelteil 10 eine schalenförmige Gleitfläche 14 auf, welche beispielsweise teilkugelförmig ausgebildet ist. Die Gleitfläche 14 wirkt mit einer Glenoidkomponente 15 zusammen, welche ebenfalls teilkugelförmig mit identischem oder zumindest ähnlichem Krümmungsradius wie die Gleitfläche 14 ausgebildet ist. Die Glenoidkomponente 15 umfaßt zumindest einen Zapfen 16 zur Verankerung im Schulterblatt (Scapula). Die Glenoidkomponente 15 ist nicht näher dargestellt, da ihre Form und insbesondere die Art der Verankerung nicht auf die dargestellte Form begrenzt ist.

Die Problematik von Schultergelenk-Endoprothesen 1 ist die geringe Überdeckung der Gelenkkugel durch die Gelenkpfanne. Dies schafft eine komplizierte Situation zur Wiederherstellung der Gelenksituation entweder nach einer Fraktur oder zur prothetischen Versorgung eines durch Arthrose geschädigten Gelenks. Problematisch sind dabei insbesondere massive Defekte an der Rotatorenmanschette (sog. Rotatorenmanschetten-Insuffizienz) verbunden mit erheblichem Funktionsverlust. Die Funktion des Gelenks wird in der Hauptsache nur durch den Delta-Muskel aufrechterhalten. Die bekannte Inversion von Gelenkkugel und Gelenkpfanne zur Vermeidung von Immobilität behebt diese Problematik relativ effizient, da sich dadurch der Drehpunkt des Gelenks nach medial verlagert, der Hebelarm verlängert und das Drehmoment erhöht wird. Dies führt zu einer signifikanten Mobilitätsverbesserung.

Nachteilig an bekannten Schultergelenk-Endoprothesen 1 ist dabei jedoch die mehrteilige Ausbildung bzw. die in den überwiegenden Fällen lösbare Verbindung zwischen der Gleitfläche des Gelenks und dem Träger dieser Gleitfläche. Die meist aus einem Polyethylen-Werkstoff bestehende Gleitfläche wird in Form eines Inlays in eine dafür vorgesehene Aufnahme eingelegt. Dies ist für den Operateur aufwendig, da zusätzliche Operationsschritte nötig sind, wodurch sich die Operationsdauer verlängert. Ein weiterer Nachteil liegt in der Fehleranfälligkeit des Inlays, welches sich in situ aus seiner Aufnahme lösen und zu Fehlfunktionen des versorgten Gelenks mit der Notwendigkeit weiterer Operationen führen kann. Die Lagerhaltung ist ebenfalls aufwendiger, da die einzelnen Komponenten bevorratet werden müssen. Der Werkstoff Polyethylen als Gleitpartner ist aufgrund seiner Verschleißanfälligkeit und häufig beobachteten aseptischen Lockerungen kritisch zu sehen.

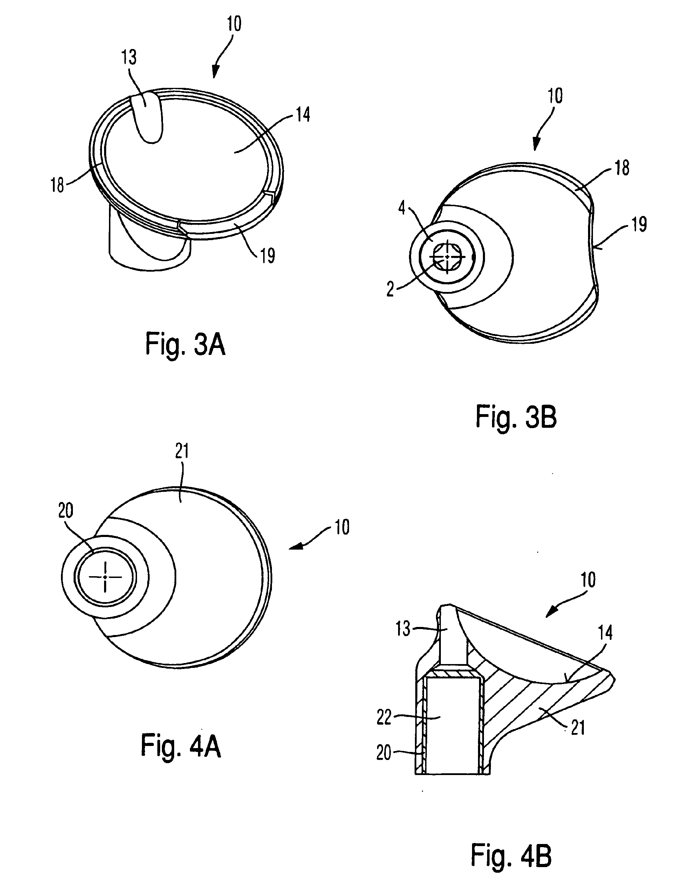

Erfindungsgemäß ist daher das Mittelteil 10, wie insbesondere aus Fig. 1C ersichtlich, einstückig ausgebildet. Alternativ kann das Mittelteil, wie in den Fig. 4A und 4B dargestellt, auch aus einem Träger 20 mit einer Gleitfläche 14 hergestellt sein, die in Form einer Ummantelung 21 nicht-lösbar mit dem Träger 20 verbunden ist, wie weiter unten beschrieben.

Dadurch können zusätzliche Komponenten wie die Polyethylen- (PE)-Inlays entfallen, die Operationsdauer verkürzt sich, die Lagerhaltung wird weniger aufwendig, und die PE-Inlays können nicht mehr aus ihrer Aufnahme herausfallen.

Das Mittelteil 10 besteht dabei vorzugsweise aus Polyetheretherketon (PEEK), einem biokompatiblen Werkstoff, welcher einfach zu verarbeiten und insbesondere auch zur Anwendung im Spritzgußverfahren geeignet sowie gegenüber Polyethylen signifikant verschleißresistenter ist.

Die Glenoidkomponente 15 besteht vorzugsweise aus Kobalt-Chrom-Legierung(CoCr), welche eine sehr glatte, widerstandsfähige Oberfläche bildet und daher hervorragende Gleiteigenschaften auf einer aus Polyetheretherketon (PEEK) gefertigten Gleitfläche 14 hat. Die Glenoidkomponente 15 kann ebenfalls aus PEEK bestehen.

Bisher bekannte inverse Schultergelenk-Endoprothesen sind vorwiegend als Primärimplantate konzipiert. D.h., es muß immer der spezielle Schaft 2 des inversen Systems neu implantiert werden, auch bei Revisionsoperationen mit noch korrekt festsitzendem Schaft 2 der ursprünglichen Hemiprothese.

Das erfindungsgemäß ausgestaltete Mittelteil 10 kann demgegenüber ausgehend von einer bereits vorhandenen Schultergelenk-Endoprothese 1 auf den in der Markhöhle des Humerus verbleibenden Schaft 2 aufgesetzt werden, ohne daß eine Totaloperation notwendig ist. Besteht z.B. nach einer Fraktur die Notwendigkeit der Versorgung mit einer Teilprothese, welche nicht invers ausgebildet ist, und treten nachfolgende Krankheitsbilder wie z.B. große Rotatorenmanschettendefekte im Schultergelenk auf, die eine weitergehende Versorgung notwendig macht, kann die vorhandene Komponente in einfacher Weise ausgetauscht werden. Der komplikationsbehaftete Ausbau des üblicherweise sehr festsitzenden Schafts 2 wird dadurch vermieden, patientenschonend gearbeitet, die Operationsdauer deutlich verkürzt sowie die Kosten für das Implantat durch Einsparung eines neuen Schafts 2 gesenkt.

Die Schultergelenk-Endoprothese 1 kann weitere Merkmale aufweisen, welche der Verbesserung der Handhabung sowie der Zuverlässigkeit dienen. So kann beispielsweise eine Entlüftungsbohrung 17 im Schaft 2 distal unterhalb des radial verbreiterten Bereichs 4 ausgebildet sein, durch welche die Ausnehmung 5 entlüftet werden kann. Dadurch können Wundsekret, Blut etc. beim Verspreizen der Komponenten durch Einschrauben des Einsatzes 7 aus der Ausnehmung 5 abgeleitet werden, so daß kein Überdruck entsteht.

Weiterhin kann eine Verdrehsicherung vorgesehen sein, welche das Verdrehen des Einsatzes 7 gegenüber dem Mittelteil 10 verhindert.

Weiterhin kann das Mittelteil 10 im Bereich eines Randes 18 eine abgerundete Kontur besitzen, um einen größeren Artikulationsbereich des prothetisch versorgten Schultergelenks zu ermöglichen.

Der Artikulationsbereich des mit einer erfindungsgemäßen Schultergelenk-Endoprothese 1 versorgten Schultergelenks kann weiterhin durch die in Fig. 3A und 3B schematisch dargestellten erfindungsgemäßen Maßnahmen gemäß einem zweiten Ausführungsbeispiel eines erfindungsgemäßen Mittelteils 10 erweitert werden.

Der Randbereich 18 des Mittelteils 10 kann zumindest einen, mehr oder weniger breiten Ausbruch 19 aufweisen, welcher der Montagebohrung 13 gegenüberliegend angeordnet ist. Ohne den Ausbruch 19 kann der Randbereich 18 des Mittelteils 10 an dem die Glenoidkomponente 15 tragenden Knochen anstehen. Durch den Ausbruch 19 kann das sogenannte "inferior notching" mit dem daraus resultierenden Verschleiß am Knochen und am Rand 18 des Mittelteils 10 zumindest für die häufigste Armposition, die Neutralposition des herabhängenden Armes, vermindert oder gänzlich vermieden werden.

Alternativ kann das Mittelteil 10 auch aus einem Träger 20 bestehen, wie in den Fig. 4A und 4B in einer Ansicht von distal und einer Schnittdarstellung eines dritten Ausführungsbeispiels eines erfindungsgemäß ausgestalteten Mittelteils 10 dargestellt, welcher beispielsweise aus Titan gefertigt ist, und mit einer Ummantelung 21 aus PEEK versehen ist. Weitere Materialpaarungen sind ebenfalls möglich und denkbar, beispielsweise Kobalt-Chrom-Legierungen (CoCr) oder keramische Materialien.

Die nicht lösbare Verbindung der Ummantelung 21 und des Trägers 20 kann dabei formschlüssig mittels Spritzgießens oder einer Schnappverbindung oder kraftschlüssig beispielsweise mittels einer Konussteckverbindung oder durch einen Kombination von form- und kraftschlüssigen Elementen erfolgen.

Der Träger 20 ist dabei konisch oder zylindrisch geformt und weist eine Ausnehmung 22 auf, welche den verbreiterten Bereich 4 des Schafts 2 in gleicher Weise aufnimmt wie die Ausnehmung 12 in dem einstückig ausgebildeten Mittelteil 10.

Das dritte Ausführungsbeispiel gemäß den Fig. 4A und 4B kann im übrigen weitere Merkmale der in den übrigen Figuren dargestellten Ausführungsbeispiele wie z.B. den Ausbruch 19 gemäß Fig. 3A und 3B aufweisen.

Die Erfindung ist nicht auf die dargestellten Ausführungsbeispiele beschränkt, sondern auch für weitere Materialkombinationen und Ausgestaltungsvarianten der einzelnen Komponenten der Schultergelenk-Endoprothese 1 geeignet. Die Erfindung ist nur durch die Ansprüche begrenzt.

## Patentansprüche

1. Schultergelenk-Endoprothese (1), umfassend einen in einer Markhöhle eines Oberarmknochens verankerbaren Schaft (2), ein mit dem Schaft (2) lösbar verbindbares Mittelteil (10) mit einer Gleitfläche (14) und eine mit der Gleitfläche (14) zu einer kugeligen Gelenkverbindung zusammenwirkenden Glenoidkomponente (15), wobei das Mittelteil (10) einstückig mit der Gleitfläche (14) hergestellt ist,
**dadurch gekennzeichnet,**
**dass** das Mittelteil (10) eine Ausnehmung (12) aufweist, dass der Schaft (2) mit einem radial verbreiterten Bereich (4) in die Ausnehmung (12) des Mittelteils (10) einschiebbar ist
und **dass** der radial verbreiterte Bereich (4) über den Umfang des Bereichs (4) verteilt Schlitze (6) aufweist.

2. Schultergelenk-Endoprothese nach Anspruch 1,
**dadurch gekennzeichnet,**
**dass** das Mittelteil (10) aus einem Träger (20) besteht, auf welchen ein die Gleitfläche (14) bildender Werkstoff nicht-lösbar aufgebracht ist.

3. Schultergelenk-Endoprothese nach Anspruch 2,
**dadurch gekennzeichnet,**
**dass** der Werkstoff ein biokompatibler Werkstoff ist.

4. Schultergelenk-Endoprothese nach einem der Ansprüche 2 oder 3,
**dadurch gekennzeichnet,**
**dass** der Werkstoff Polyetheretherketon (PEEK) ist.

5. Schultergelenk-Endoprothese nach einem der Ansprüche 2 bis 4,
**dadurch gekennzeichnet,**
**dass** der Werkstoff in Form einer Ummantelung (21) auf dem Träger (20) aufgebracht ist.

6. Schultergelenk-Endoprothese nach Anspruch 5,
**dadurch gekennzeichnet,**
**dass** das Mittelteil (10) durch ein Spritzgussverfahren durch Umspritzen des Trägers (20) mit der Ummantelung (21) hergestellt ist.

7. Schultergelenk-Endoprothese nach Anspruch 5 oder 6,
**dadurch gekennzeichnet,**
**dass** der die Ummantelung (21) bildende und die Gleitfläche (14) umfassende Werkstoff durch Kraftschluß oder durch eine Kombination aus Kraft- und Formschluß mit dem Träger (20) nicht-lösbar verbunden ist.

8. Schultergelenk-Endoprothese nach einem der Ansprüche 2 bis 7,
**dadurch gekennzeichnet,**
**dass** der Träger (20) hülsenförmig ausgebildet ist.

9. Schultergelenk-Endoprothese nach einem der Ansprüche 2 bis 8,
**dadurch gekennzeichnet,**
**dass** der hülsenförmige Träger (20) eine zylindrische oder konische Außenkontur aufweist.

10. Schultergelenk-Endoprothese nach einem der Ansprüche 2 bis 9,
**dadurch gekennzeichnet,**
**dass** der Träger (20) aus Titan oder einem anderen geeigneten Material ausgebildet ist.

11. Schultergelenk-Endoprothese nach Anspruch 1 oder 2,
**dadurch gekennzeichnet,**
**dass** das Mittelteil (10) einteilig durch ein Spritzgussverfahren hergestellt ist.

12. Schultergelenk-Endoprothese nach einem der Ansprüche 2 bis 11,
**dadurch gekennzeichnet,**
**dass** der Werkstoff eine Kobalt-Chrom-Legierung (CoCr) ist.

13. Schultergelenk-Endoprothese nach einem der Ansprüche 1 bis 12,
**dadurch gekennzeichnet,**
**dass** die Gleitfläche (14) des Mittelteils (10) poliert ist.

14. Schultergelenk-Endoprothese nach einem der Ansprüche 1 bis 13,
**dadurch gekennzeichnet,**
**dass** die Glenoidkomponente (15) aus einer Kobalt-Chrom-Legierung (CoCr) hergestellt ist.

15. Schultergelenk-Endoprothese nach einem der Ansprüche 2 bis 14,
**dadurch gekennzeichnet,**
**dass** das Mittelteil (10) eine Ausnehmung (12) aufweist.

16. Schultergelenk-Endoprothese nach Anspruch 1 und Anspruch 15,
**dadurch gekennzeichnet,**
**dass** sich die Ausnehmung (12) von einem distalen Ende des Mittelteils (10) nach proximal achsparallel zu einer Längsachse (11) des Schafts (2) erstreckt.

17. Schultergelenk-Endoprothese nach Anspruch 1 und Anspruch 15 oder 16,
**dadurch gekennzeichnet,**
**dass** sich die Ausnehmung (12) innerhalb des Mittelteils (10) zu einer Montagebohrung (13) verjüngt.

18. Schultergelenk-Endoprothese nach Anspruch 17,
**dadurch gekennzeichnet,**
**dass** die Montagebohrung (13) proximal aus dem Mittelteil (10) ausmündet.

19. Schultergelenk-Endoprothese nach einem der Ansprüche 15 bis 18,
**dadurch gekennzeichnet,**
**dass** das Mittelteil (10) gekröpft relativ zu einer Längsachse (11) des Schaftes (2) ausgebildet ist.

20. Schultergelenk-Endoprothese nach Anspruch 1 und nach einem der Ansprüche 15 bis 19,
**dadurch gekennzeichnet,**
**dass** der Schaft (2) mit einem radial verbreiterten Bereich (4) in die Ausnehmung (12) des Mittelteils (10) einschiebbar ist.

21. Schultergelenk-Endoprothese nach Anspruch 20,
**dadurch gekennzeichnet,**
**dass** der radial verbreiterte Bereich (4) über den Umfang des Bereichs (4) verteilt Schlitze (6) aufweist.

22. Schultergelenk-Endoprothese nach Anspruch 21,
**dadurch gekennzeichnet,**
**dass** der radial verbreiterte Bereich (4) in der Ausnehmung (12) des Mittelteils (10) durch Verdrehen eines Einsatzes (7) gegen einen im Querschnitt keilförmigen Ringeinsatz (9) verspreizbar ist.

23. Schultergelenk-Endoprothese nach Anspruch 22,
**dadurch gekennzeichnet,**
**dass** das Verdrehen des Einsatzes (7) in kommunizierenden Gewinden (8a, 8b), welche in einer Ausnehmung (5) des radial verbreiterten Bereichs (4) und an dem Einsatz (7) ausgebildet sind, erfolgt.

24. Schultergelenk-Endoprothese nach Anspruch 23,
**dadurch gekennzeichnet,**
**dass** das Mittelteil (10) bei einer Revisionsoperation nach Demontage eines ursprünglichen Mittelteils ohne Explantation des Schaftes (2) ersetzt werden kann.

25. Schultergelenk-Endoprothese nach einem der Ansprüche 15 bis 24,
**dadurch gekennzeichnet,**
**dass** an einem Randbereich (18) des Mittelteil (10) zumindest ein Ausbruch (19) vorgesehen ist, in welchem der Randbereich (18) abgesenkt ist.

26. Schultergelenk-Endoprothese nach Anspruch 25,
**dadurch gekennzeichnet,**
**dass** der zumindest eine Ausbruch (19) radial gegenüberliegend zu der Montagebohrung (13) angeordnet ist.

27. Schultergelenk-Endoprothese nach Anspruch 25 oder 26,
**dadurch gekennzeichnet,**
**dass** eine aus der Ausnehmung (5) distal-radial ausmündende Entlüftungsbohrung (17) vorgesehen ist.

28. Schultergelenk-Endoprothese nach einem der Ansprüche 1 bis 27,
**dadurch gekennzeichnet,**
**dass** der Schaft (2) in einer Markhöhle des Oberarmknochens verankerbar ist.

29. Schultergelenk-Endoprothese nach einem der Ansprüche 1 bis 28,
**dadurch gekennzeichnet,**
**dass** die Glenoidkomponente (15) in dem Schulterblattknochen verankerbar ist.

## Claims

1. Shoulder-joint endoprosthesis (1) comprising a shaft (2) which can be anchored in a marrow cavity in a bone of the upper arm, a central part (10) which can be detachably connected to said shaft (2) and has a sliding face (14), and a glenoid component (15) which interacts with the sliding face (14) to form a spherical joint connection, wherein the central part (10) is manufactured in one piece with the sliding face (14),
**characterised in that**
the central part (10) has a clearance (12),
that the shaft (2) can be inserted, by means of a radially widened region (4), in the clearance (12) in the central part (10),
and that the radially widened region (4) has slots (6) which are distributed over the circumference of said region (4).

2. Shoulder-joint endoprosthesis according to claim 1,
**characterised in that**
the central part (10) consists of a carrier (20) to which a material forming the sliding face (14) is applied in a non-detachable manner.

3. Shoulder-joint endoprosthesis according to claim 2,
**characterised in that**
the material is a biocompatible material.

4. Shoulder-joint endoprosthesis according to either of claims 2 or 3,
**characterised in that**
the material is polyetheretherketone (PEEK).

5. Shoulder-joint endoprosthesis according to one of claims 2 to 4,
**characterised in that**
the material is applied in the form of a sheathing (21) on the carrier (20).

6. Shoulder-joint endoprosthesis according to claim 5,
**characterised in that**
the central part (10) is manufactured by an injection-moulding process by overmoulding the carrier (20) with the sheathing (21).

7. Shoulder-joint endoprosthesis according to claim 5 or 6,
**characterised in that**
the material which forms the sheathing (21) and comprises the sliding face (14) is connected to the carrier (20) in a non-detachable manner by force-locking or by a combination of force-locking and form-locking.

8. Shoulder-joint endoprosthesis according to one of claims 2 to 7,
**characterised in that**
the carrier (20) is of sleeve-shaped construction.

9. Shoulder-joint endoprosthesis according to one of claims 2 to 8,
**characterised in that**
the sleeve-shaped carrier (20) has a cylindrical or conical outer contour.

10. Shoulder-joint endoprosthesis according to one of claims 2 to 9,
**characterised in that**
the carrier (20) is constructed from titanium or other suitable material.

11. Shoulder-joint endoprosthesis according to claim 1 or 2,
**characterised in that**
the central part (10) is manufactured in one piece by an injection-moulding process.

12. Shoulder-joint endoprosthesis according to one of claims 2 to 11,
**characterised in that**
the material is a cobalt/chromium (CoCr) alloy.

13. Shoulder-joint endoprosthesis according to one of claims 1 to 12,
**characterised in that**
the sliding face (14) of the central part (10) is polished.

14. Shoulder-joint endoprosthesis according to one of claims 1 to 13,
**characterised in that**
the glenoid component (15) is manufactured from a cobalt/chromium (CoCr) alloy.

15. Shoulder-joint endoprosthesis according to one of claims 2 to 14,
**characterised in that**
the central part (10) has a clearance (12).

16. Shoulder-joint endoprosthesis according to claims 1 and 15,
**characterised in that**
the clearance (12) extends from a distal end of the central part (10) in the proximal direction, in a manner axially parallel to a longitudinal axis (11) of the shaft (2).

17. Shoulder-joint endoprosthesis according to claim 1 and claim 15 or 16,
**characterised in that**
the clearance (12) tapers, inside the central part (10), to form a mounting bore (13).

18. Shoulder-joint endoprosthesis according to claim 17,
**characterised in that**
the mounting bore (13) emerges proximally from the central part (10).

19. Shoulder-joint endoprosthesis according to one of claims 15 to 18,
**characterised in that**
the central part (10) is constructed so as to be offset relative to a longitudinal axis (11) of the shaft (12).

20. Shoulder-joint endoprosthesis according to claim 1 and one of claims 15 to 19,
**characterised in that**
the shaft (2) can be inserted, by means of a radially widened region (4), in the clearance (12) in the central part (10).

21. Shoulder-joint endoprosthesis according to claim 20,
**characterised in that**
the radially widened region (4) has slots (6) which are distributed over the circumference of said region (4) .

22. Shoulder-joint endoprosthesis according to claim 21,
**characterised in that**
the radially widened region (4) can be braced, within the clearance (12) in the central part (10), against an annular insert (9) which is wedge-shaped in cross-section, by twisting an insert (7).

23. Shoulder-joint endoprosthesis according to claim 22,
**characterised in that**
the twisting of the insert (7) takes place within communicating threads (8a, 8b) which are constructed in a clearance (5) in the radially widened region (4) and on the insert (7).

24. Shoulder-joint endoprosthesis according to claim 23,
**characterised in that**
the central part (10) can be replaced in the course of an inspecting operation after the demounting of an original central part without explanting the shaft (2).

25. Shoulder-joint endoprosthesis according to one of claims 15 to 24,
**characterised in that**
there is provided, at an edge region (18) of the central part (10), at least one excavation (19) within which said edge region (18) is lowered.

26. Shoulder-joint endoprosthesis according to claim 25,
**characterised in that**
the at least one excavation (19) is disposed in a radially opposite manner in relation to the mounting bore (13).

27. Shoulder-joint endoprosthesis according to claim 25 or 26,
**characterised in that**
a ventilating bore (17) which emerges distally/radially from the clearance (5) is provided.

28. Shoulder-joint endoprosthesis according to one of claims 1 to 27,
**characterised in that**
the shaft (2) can be anchored in a marrow cavity in the bone of the upper arm.

29. Shoulder-joint endoprosthesis according to one of claims 1 to 28,
**characterised in that**
the glenoid component (15) can be anchored in the bone of the scapula.

## Revendications

1. Endoprothèse de l'articulation de l'épaule (1), comprenant une tige (2) pouvant être ancrée dans une cavité médullaire d'un humérus, un élément médian (10) pouvant être relié de façon amovible à la tige (2) avec une surface de glissement (14) et un composant glénoïde (15) coopérant avec la surface de glissement (14) sous forme de liaison articulée sphérique, l'élément médian (10) étant fabriqué d'un seul tenant avec la surface de glissement (14),
**caractérisée en ce**
**que** l'élément médian (10) comprend un évidement (12), en ce que la tige (2) ayant un secteur élargi de façon radiale (4) peut être insérée dans l'évidement (12) de l'élément médian (10)
et en ce que le secteur élargi de façon radiale (4) comprend des rainures (6) réparties sur la périphérie du secteur (4).

2. Endoprothèse de l'articulation de l'épaule selon la revendication 1,
**caractérisée en ce**
**que** l'élément médian (10) se compose d'un support (20), sur lequel un matériau formant la surface de glissement (14) est appliqué de façon non amovible.

3. Endoprothèse de l'articulation de l'épaule selon la revendication 2,
**caractérisée en ce**
**que** le matériau est un matériau biocompatible.

4. Endoprothèse de l'articulation de l'épaule selon l'une quelconque des revendications 2 ou 3,
**caractérisée en ce**
**que** le matériau est du polyéther éther cétone (PEEK).

5. Endoprothèse de l'articulation de l'épaule selon l'une quelconque des revendications 2 à 4,
**caractérisée en ce**
**que** le matériau est appliqué sous la forme d'un enrobage (21) sur le support (20).

6. Endoprothèse de l'articulation de l'épaule selon la revendication 5,
**caractérisée en ce**
**que** l'élément médian (10) est fabriqué via un procédé de moulage par injection via un enrobage par injection du support (20) avec l'enrobage (21).

7. Endoprothèse de l'articulation de l'épaule selon la revendication 5 ou 6,
**caractérisée en ce**
**que** le matériau formant l'enrobage (21) et entourant la surface de glissement (14) est relié au support (20) de façon non amovible par adhérence ou via une combinaison par adhérence et concordance de forme.

8. Endoprothèse de l'articulation de l'épaule selon l'une quelconque des revendications 2 à 7,
**caractérisée en ce**
**que** le support (20) est conçu en forme de douille.

9. Endoprothèse de l'articulation de l'épaule selon l'une quelconque des revendications 2 à 8,
**caractérisée en ce**
**que** le support (20) en forme de douille comprend un contour extérieur cylindrique ou conique.

10. Endoprothèse de l'articulation de l'épaule selon l'une quelconque des revendications 2 à 9,
**caractérisée en ce**
**que** le support (20) est conçu à base de titane ou d'un autre matériau approprié.

11. Endoprothèse de l'articulation de l'épaule selon la revendication 1 ou 2,
**caractérisée en ce**
**que** l'élément médian (10) est fabriqué d'un seul tenant via un procédé de moulage par injection.

12. Endoprothèse de l'articulation de l'épaule selon l'une quelconque des revendications 2 à 11,
**caractérisée en ce**
**que** le matériau est un alliage de chrome et de cobalt (CoCr).

13. Endoprothèse de l'articulation de l'épaule selon l'une quelconque des revendications 1 à 12,
**caractérisée en ce**
**que** la surface de glissement (14) de l'élément médian (10) est polie.

14. Endoprothèse de l'articulation de l'épaule selon l'une quelconque des revendications 1 à 13,
**caractérisée en ce**
**que** le composant glénoïde (15) est fabriqué à partir d'un alliage de chrome et de cobalt (CoCr).

15. Endoprothèse de l'articulation de l'épaule selon l'une quelconque des revendications 2 à 14,
**caractérisée en ce**
**que** l'élément médian (10) comprend un évidement (12).

16. Endoprothèse de l'articulation de l'épaule selon la revendication 1 et la revendication 15,
**caractérisée en ce**
**que** l'évidement (12) s'étend d'une extrémité distale de l'élément médian (10) suivant un axe proximal parallèle à un axe longitudinal (11) de la tige (2).

17. Endoprothèse de l'articulation de l'épaule selon la revendication 1 et la revendication 15 ou 16,
**caractérisée en ce**
**que** l'évidement (12) s'effile dans l'élément médian (10) jusqu'à un alésage de montage (13).

18. Endoprothèse de l'articulation de l'épaule selon la revendication 17,
**caractérisée en ce**
**que** l'alésage de montage (13) débouche au niveau proximal de l'élément médian (10).

19. Endoprothèse de l'articulation de l'épaule selon l'une quelconque des revendications 15 à 18,
**caractérisée en ce**
**que** l'élément médian (10) est conçu de façon contre-coudée par rapport à un axe longitudinal (11) de la tige (2).

20. Endoprothèse de l'articulation de l'épaule selon la revendication 1 et selon l'une quelconque des revendications 15 à 19,
**caractérisée en ce**
**que** la tige (2) peut être insérée avec un secteur élargi de façon radiale (4) dans l'évidement (12) de l'élément médian (10).

21. Endoprothèse de l'articulation de l'épaule selon la revendication 20,
**caractérisée en ce**
**que** le secteur élargi de façon radiale (4) comprend des rainures (6) réparties sur la périphérie du secteur (4).

22. Endoprothèse de l'articulation de l'épaule selon la revendication 21,
**caractérisée en ce**
**que** le secteur élargi de façon radiale (4) peut être moulé par injection dans l'évidement (12) de l'élément médian (10) via la torsion d'un insert (7) contre un insert annulaire (9) cunéiforme dans la coupe transversale.

23. Endoprothèse de l'articulation de l'épaule selon la revendication 22,
**caractérisée en ce**
**que** la torsion de l'insert (7) s'effectue dans des filetages communiquant (8a, 8b) qui sont formés dans un évidement (5) du secteur élargi de façon radiale (4) et au niveau de l'insert (7).

24. Endoprothèse de l'articulation de l'épaule selon la revendication 23,
**caractérisée en ce**
**que** l'élément médian (10) peut être remplacé dans le cas d'une opération de révision après le démontage d'un élément médian d'origine sans explantation de la tige (2).

25. Endoprothèse de l'articulation de l'épaule selon l'une quelconque des revendications 15 à 24,
**caractérisée en ce**
**qu'**au moins un creux (19) est prévu au niveau d'un secteur marginal (18) de l'élément médian (10) dans lequel le secteur marginal (18) est rabattu.

26. Endoprothèse de l'articulation de l'épaule selon la revendication 25,
**caractérisée en ce**
**qu'**au moins un creux (19) est disposé de façon radiale opposée à l'alésage de montage (13).

27. Endoprothèse de l'articulation de l'épaule selon la revendication 25 ou 26,
**caractérisée en ce**
**qu'**un orifice de purge d'air (17) débouchant de façon distale-radiale hors de l'évidement (5) est prévu.

28. Endoprothèse de l'articulation de l'épaule selon l'une quelconque des revendications 1 à 27,
**caractérisée en ce**
**que** la tige (2) peut être ancrée dans une cavité médullaire d'un humérus.

29. Endoprothèse de l'articulation de l'épaule selon l'une quelconque des revendications 1 à 28,
**caractérisée en ce**
**que** le composant glénoïde (15) peut être ancré dans l'os de l'omoplate.
